# EUROPÄISCHE PATENTSCHRIFT

(11) **EP 0 915 945 B1**
(45) Veröffentlichungstag und Bekanntmachung des Hinweises auf die Patenterteilung: **11.10.2000**
(21) Anmeldenummer: 97915370.7
(22) Anmeldetag: 14.03.1997
(51) Int. Cl.: C11D 1/28, C11D 1/10, C11D 1/34, A61K 7/50

(54) **FORMULIERUNGEN FÜR WASCH-, REINIGUNGS- UND KÖRPERPFLEGEMITTEL ENTHALTEND ANIONISCHE GEMINITENSIDE UND IHRE VERWENDUNG**
FORMULATIONS FOR WASHING, CLEANING AND BODY CARE AGENTS COMPRISING ANIONIC GEMINI SURFACTANTS AND THEIR USE
FORMULATIONS POUR DETERGENTS, NETTOYANTS ET AGENTS DE SOINS CORPORELS COMPRENANT DES TENSIOACTIFS ANIONIQUES ET LEUR UTILISATION

(30) Priorität: 23.04.1996 DE 19616096
(43) Veröffentlichungstag der Anmeldung: 19.05.1999
(73) Patentinhaber: RWE-DEA Aktiengesellschaft für Mineraloel und Chemie, 22297 Hamburg (DE)
(72) Erfinder: KWETKAT, Klaus, D-44534 Lünen (DE); BROCK, Michael, D-46514 Schermbeck (DE); KOCH, Herbert, D-46282 Dorsten (DE)
(74) Vertreter: Schupfner, Gerhard D., Dr. Dipl.-Chem.
(86) Internationale Anmeldenummer: EP9701298
(87) Internationale Veröffentlichungsnummer: WO9740124

(56) Entgegenhaltungen:
- WO-A-95/19955
- DE-A- 4 440 328

## Beschreibung

Gegenstand der Erfindung ist die Verwendung von anionischen Geminitensiden, gemäß der Formel (I) in Formulierungen für das Waschen und Reinigen von Textilien, das Reinigen von harten Oberflächen und das Reinigen und Waschen von Haut und Haar, sowie Wasch-, Reinigungs- und Körperpflegemittel, welche mindestens 0,1 Gew.-% der anionischen Geminitenside der Formel (I) enthalten.

Die Zusammenstellung von Formulierungen für Wasch-, Reinigungs- und Körperpflegemittel ist eine komplexe Aufgabe, da die Formulierungen in der Lage sein müssen, Verschmutzungen der verschiedensten Art von sehr unterschiedlichen Oberflächen zu entfernen. Besonders die schnelle und effiziente Entfernung von fettigen oder öligen Anschmutzungen ist im allgemeinen problematisch. Neben den ausschließlich reinigungstechnischen Anforderungen werden die ökotoxikologischen Anforderungen an Formulierungen für Wasch-, Reinigungs- und Körperpflegemittel immer stringenter.

Zur Schonung der natürlichen Ressourcen gehört jedoch nicht allein die Verwendung von Tensiden auf Basis nachwachsender Rohstoffe, sondern ganz besonders auch die Herstellung von bei gleichbleibendem Rohstoffeinsatz immer wirksameren Formulierungen, die trotzdem den Anforderungen an ihre biologische Abbaubarkeit und Hautmildheit genügen. Darüber hinaus müssen die immer kompakter werdenden tensidhaltigen Formulierungen für Wasch- und Reinigungsmittel auch bei ebenfalls aus ökologischen Gründen sinkender Wassermenge in der Waschflotte schnell in Wasser löslich sein.

All diese Anforderungen lassen sich nicht mehr allein auf physikalischem Wege erfüllen, sondern erfordern den Einsatz leistungsfähigerer Tenside. Die als "New Generation of Surfactants" bezeichneten Gemini- oder auch Zwillingstenside (M. J. Rosen, Chemtech, No. 3 (1995) 30) sind, sofern ihre Struktur optimal gewählt ist, Tenside mit einer deutlich höheren Leistungsfähigkeit als ihre konventionellen Äquivalente und bieten darüber hinaus, bei der Wahl der richtigen Strukturvariante, eine hohe Multifunktionalität und helfen somit, die Wasch- bzw. Reinigungsleistung pro Masseneinheit der Formulierung zu steigern.

Bei den in den Anmeldungen WO 95/19953 und WO 95/19955 beschriebenen Geminipolyhydroxyfettsäure- und Geminipolyetherfettsäureamiden handelt es sich um nichtionische Geminitenside. Im Vergleich zu den heute bekannten nichtionischen Tensiden bieten diese Verbindungen jedoch keine besondere Steigerung der Effizienz pro Einsatzmasse. Auch kann die Multifunktionalität dieser Verbindungen nur durch eine deutliche Steigerung der Molekülmasse erzielt werden, was hinsichtlich der Schonung des Ökosystems als eher kontraproduktiv betrachtet werden muß.

Werden jedoch anionische Geminitenside, wie sie in DE-A 44 40 328 beschrieben sind (Formel I), eingesetzt, kommt man zu einer signifikanten Effizienzsteigerung gesamter Endformulierungen. Das ist möglich, weil die anionischen Geminitenside deutlich effizienter als konventionelle anionische Tenside hinsichtlich beispielsweise der kritischen Mizellbildungskonzentration, Grenzflächenspannung, Wasserlöslichkeit, Härtestabilität, lösungsvermittelnder Wirkung und Waschkraft und darüber hinaus aufgrund ihrer besonderen Struktur besonders hautmild und biologisch abbaubar sind. 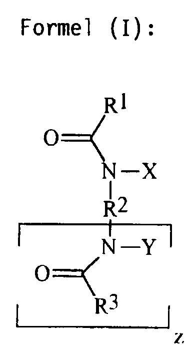 Gegenstand der Erfindung ist daher die Verwendung von mindestens 0, 1 % anionischen Geminitensiden gemäß der Formel (I), in Wasch-, Reinigungs- und Körperpflegemitteln, wobei R¹ und R³ unabhängig voneinander je für einen unverzweigten oder verzweigten, gesättigten oder ungesättigten Kohlenwasserstoffrest mit 1 bis 22, vorzugsweise 7 bis 17 Kohlenstoffatomen, und R² für einen Spacer aus einer unverzweigten oder verzweigten Kette mit 2 bis 100 Kohlenstoffatomen, die 0 bis 20 Sauerstoff-, 0 bis 20 Stickstoff-, 0 bis 4 Schwefel- und/oder 0 bis 3 Phosphoratome enthält und die 0 bis 20 funktionelle Seitengruppen, wie z. B. Hydroxyl-, Carbonyl-, Carboxyl-, Amino- und/oder Acylaminogruppen, aufweist und der 0 bis 20, Alkoxygruppen enthält, steht, X und Y unabhängig voneinander funktionelle Gruppen bedeuten und der Substitutionsgrad z = 1 bis 10 beträgt. Eine ausführliche Beschreibung verschiedener Ausführungen des Spacers ist in der Deutschen Patentanmeldung 44 40 328 enthalten, die wir hiermit als Referenz anführen.

X oder Y steht für einen Substituenten der Formel II

-(C₂H₄O)_{α} (C₃H₆O)_{β}H (II)

mit α = 0 bis 50, vorzugsweise α = 10 bis 30,
β = 0 bis 60, vorzugsweise β = 20 bis 40,
und α + β = 1 bis 100, vorzugsweise α + β = 10 bis 50,
und X oder Y für einen Substituenten der Formel III,
oder X oder Y stehen unabhängig voneinander für Substituenten der Formel III

-(C₂H₄O)_{γ}(C₃H₆O)_{δ}-FR (III)

mit jeweils γ = 0 bis 20, vorzugsweise γ = 0 bis 8,
δ = 0 bis 20, vorzugsweise δ = 0 bis 12,
und γ + δ = 1 bis 40, vorzugsweise γ + δ = 5 bis 20,
wobei die Alkoxideinheiten statistisch oder blockweise eingebunden sind und die Reihenfolge beliebig ist,
und wobei FR für einen funktionellen Rest -CH₂-COOM, -SO₃M, -P(O)(OM)₂, -C₂H₄-SO₃M oder -O-C(O)-C₂H₃(SO₃M)-CO₂M'
mit M, M' = Alkali-, Ammonium-, Alkanolammonium- oder ½ Erdalkaliion steht.

Der Alkoxylierungsgrad ist jeweils ein Mittelwert und kann jeden beliebigen, auch nicht ganzzahligen Wert in den angegebenen Grenzen annehmen.

Neben mindestens 0,1 % anionischem Geminitensid gemäß der Formel (I), das als Mischung verschiedener Homologen der gleichen Grundstruktur (sowohl hinsichtlich der C-Kettenlänge der lipophilen Ketten als auch des Spacers oder der Alkoxyetherketten) und Mischung von Verbindungen mit verschiedenem Funktionalisierungsgrad, zum Beispiel Sulfierungsgrad zwischen 1 und 2, vorliegen kann, können die erfindungsgemäßen Formulierungen für Wasch-, Reinigungs- und Körperpflegemittel noch jeweils wenigstens 0,001, vorzugsweise wenigstens 0,1 % einer oder mehrerer Wasch-, Reinigungs- oder Körperpflegemittelkomponenten, die im folgenden näher erläutert werden, enthalten. Alle Prozentangaben sind als Gewichtsprozente zu verstehen, es sei denn, die Prozentangabe wird explizit auf eine andere Größe bezogen. Als solche Komponenten sind die folgenden zu nennen:
a) Enzyme: Eine ganze Reihe von Enzymen können in den erfindungsgemäßen Formulierungen enthalten sein, so zum Beispiel Proteasen, Amylasen, Lipasen, Cellulasen und Peroxidasen sowie Mischungen der jeweiligen Enzyme. Auch andere Enzyme können in die Waschmittelformulierungen eingearbeitet werden, wobei sie, wie die vorgenannten, von verschiedenster Herkunft aus Bakterien, Pilzen, z. B. Hefepilzen, und anderen Pflanzen stammen, aber auch tierischen Ursprungs sein können.

Unterschiedliche Faktoren bestimmen die Auswahl einzelner Enzyme, wie beispielsweise die pH-Aktivitäts- und / oder -Stabilitätsoptima, die Thermostabilität, die Stabilität gegenüber verschiedenen Tensiden, Buildern usw. Enzyme werden in Einwaagen bis zu 50 mg, bevorzugt 0,01 mg bis 3 mg aktives Enzym auf ein Gramm Waschmittel formulierung eingesetzt, d. h. 0,001 % bis ca. 5 % in den Waschmittelformulierungen. Für Proteasen gilt eine Einsatzkonzentration einer Aktivität von 0,005 bis 0,1 Anson Einheiten (Anson Units = AU) pro Gramm Formulierung.

b) Enzymstabilisatoren: Dazu gehören wasserlösliche Quellen von Calcium- oder/und Magnesiumionen, die häufig zugesetzt werden müssen, damit das Buildersystem nicht auch diese Zentralatome der Enzyme entfernt und sie damit desaktiviert. Calciumionen sind hier im allgemeinen effektiver als Magnesiumionen. Zusätzliche Stabilisierung kann durch den Zusatz von Boraten (z.B. Severson, US 4 537 706) erfolgen. Typischerweise enthalten die Formulierungen 1 bis 30, vorzugsweise 2 bis 20, besonders bevorzugt 5 bis 15 und ganz besonders bevorzugt 8 bis 12 Millimole Calciumionen pro Liter Endformulierung.
Obwohl die Konzentration in verschiedenen Formulierungen abhängig von den verwendeten Enzymen variieren kann, sollten immer genug Calciumionen nach der Komplexierung durch das Buildersystem und durch Seifen verfügbar sein, um die Enzyme aktiviert zu halten. Jedes wasserlösliche Calcium- oder Magnesiumsalz kann verwendet werden. Es seien hier die folgenden Beispiele, ohne die erfindungsgemäßen Formulierungen darauf einzuschränken, erwähnt: Calciumchlorid, -formiat, -sulfat, -hydroxid, -malat, - maleat, -acetat und die entsprechenden Magnesiumsalze. Abhängig von der Menge und Art der verwendeten Enzyme enthalten die Waschmittelformulierungen 0,05 % bis 2 % wasserlösliche Calcium und / oder Magnesiumsalze.
Boratstabilisatoren sind zu 0,25 % bis 10 %, bevorzugt 0,5 % bis 5 % und besonders bevorzugt 0,75 % bis 3 %, berechnet als Borsäure, in den Formulierungen enthalten. Die zugesetzten Boratstabilisatoren müssen in der Lage sein, Borsäure bilden zu können. Hier ist der direkte Einsatz von Borsäure bevorzugt, doch können auch, ohne darauf einzuschränken, Boroxid, Borax, andere Alkaliborate und substituierte Borsäuren wie z. B. Phenyl-, Butyl- und p-Bromphenylborsäure eingesetzt werden.

c) Bleichsysteme - Bleichmittel und Bleichaktivatoren: Für die erfindungsgemäßen Formulierungen ist die Verwendung eines Bleichsystems, sei es Bleichmittel und - aktivator oder lediglich ein Bleichmittel, optional. Sofern verwendet, werden die Bleichmittel in Mengen von 1 bis 30 %, bevorzugt 5 bis 20 %, eingesetzt. Sofern eingesetzt, werden Bleichaktivatoren in Mengen von 0,1 bis 60 % des Bleichmittels verwendet. Bevorzugt werden 0,5 bis 40 % Bleichsystem, bezogen auf die Formulierung. Alle für die Reinigung von Textilien, harten Oberflächen (Industrie- und Haushaltsreiniger, Geschirrspülmittel) oder andere Reinigungsaufgaben geeignete Bleichmittel können eingesetzt werden. Dazu zählen sowohl auf Sauerstoffbasis arbeitende Bleichmittel wie auch andere Systeme. Perborate, z. B. Natriumperborate, sei es als Mono- oder Tetrahydrat, können eingesetzt werden, ebenso wie Percarbonsäure-Bleichmittel und deren Salze. Zu den geeigneten Vertretern dieser Klasse zählen Magnesiumperoxyphthalat-hexahydrat, Magnesium-metachlorperbenzoat, 4-Nonylamino-4-oxoperoxybutansäure, Diperoxydodecandisäure und, besonders bevorzugt, 6-Nonylamino-6-oxoperoxycaprinsäure (Burns et al., US 4 634 551). Persauerstoffbleichmittel können ebenfalls eingesetzt werden. Zu geeigneten Vertretern dieser Klasse zählen Natriumcarbonatperoxohydrat und vergleichbare Percarbonate, Natriumpyrophosphatperoxohydrat, Harnstoffperoxohydrat, Natriumperoxid und Persulfatbleichmittel. Auch Mischungen von Bleichmitteln können in den erfindungsgemäßen Wasch- und Reinigungsmittelformulierungen eingesetzt werden.
Persauerstoffbleichmittel werden bevorzugt mit Bleichaktivatoren kombiniert, zu denen, ohne die erfindungsgemäßen Formulierungen darauf zu beschränken, Nonanoyloxy-phenylsulfonat, Tetraacetylethylendiamin und deren Mischungen sowie andere in US 4 634 551 erwähnte Kombinationen von Bleichmitteln und -aktivatoren gehören. Ganz besonders bevorzugt als Bleichaktivatoren sind Amidderivate der Formeln R¹N(R⁵C(O)R²C(O)L oder R¹C(O)N(R⁵)R²C(O)L, wobei R¹ eine Alkylgruppe mit 6 bis 12 Kohlenstoffatomen, R² eine Alkylengruppe mit 1 bis 6 Kohlenstoffatomen, R⁵ ein Wasserstoffatom oder Alkyl-, Aryl- oder Alkylaryl mit 1 bis 10 Kohlenstoffatomen und L jedwede für nucleophile Reaktionen geeignete Abgangsgruppe (z. B. Phenylsulfonat) bedeuten. Als Beispiele seien hier die folgenden Verbindungen erwähnt: (6-Octanamido-caproyl)oxyphenylsulfonat, (6-Nonanamido-caproyl)oxyphenylsulfonat, (6-Decanamidocaproyl)oxyphenylsulfonat und deren Mischungen. Acyllactamaktivatoren gehören zu einer anderen Klasse bevorzugter Bleichaktivatoren, hier besonders Acylcaprolactam und Acylvalerolactam mit Alkyl-, Aryl-, Alkoxyaryl- und Alkylaryl-acylgruppen, die 1 bis 16 Kohlenstoffatome enthalten. Unter den nicht auf Sauerstoff basierenden Bleichmitteln gehören sulfonierte Zink- und/oder Aluminium-phthalocyanine zu den bevorzugten Systemen.

d) Buildersysteme: Ebenfalls optional können die erfindungsgemäßen Wasch- und Reinigungsmittel Buildersysteme (Gesamtbuilder), die aus wasserenthärtenden Silikaten und sonstigen anorganischen und/oder organischen Buildern bestehen, enthalten. Sie werden in Waschmittelformulierungen eingesetzt, um die Partikelschmutzentfernung zu unterstützen und die Wasserhärte zu kontrollieren. Flüssigformulierungen enthalten 0 bis 50 %, bevorzugt 5 bis 50 % und besonders bevorzugt 5 bis 30 % Gesamtbuilder. Granulierte Formulierungen enthalten 0 bis 80 %, bevorzugt 10 bis 80 % und besonders bevorzugt 15 bis 50 % Gesamtbuilder. Höhere Konzentrationen sollen hier jedoch nicht ausgeschlossen werden.

Zu den anorganischen Buildern zählen insbesondere Silikate und Alumosilikate.
Beispiele für Silikatbuilder sind Alkalisilikate, besonders solche mit SiO₂ : Na₂O im Verhältnis 1,6 : 1 bis 3,2 : 1 und Schichtsilikate wie Natriumsilikate vom Typ NaMSiₓO₂ₓ₊₁ ·yH₂O (M steht für Na oder H, x = 1,9 - 4, y = 0-20). Besonders bevorzugt ist der mit SKS-6 bezeichnete Typ. Auch Magnesiumsilikate können hier eingesetzt werden. Alumosilikate sind ebenfalls nützlich in den erfindungsgemäßen Formulierungen und besonders wichtig in granularen Waschmittelformulierungen. Die verwendbaren Alumosilikatbuilder können mit der empirischen Formel [M_{z}(zAlO₂)_{y}]·xH₂O beschrieben werden, z und y nehmen Werte von wenigstens 6 an , das molare Verhältnis von z zu y liegt im Bereich von 1,0 bis 0,5, x nimmt Werte von ca. 0 bis 30 an. Es kann sich sowohl um kristalline als auch um amorphe, synthetische oder natürlich vorkommende Alumosilikate handeln. Die Silikatbuilder können zu 0 bis 60 % enthalten sein.

Zu den anorganischen Buildern zählen weiterhin, ohne die Formulierungen darauf einzuschränken, Alkali-, Ammonium- und Alkanolammoniumsalze von Polyphosphaten (z. B. Tripolyphosphate, Pyrophosphate und polymere Metaphosphate), Phosphonate, Carbonate (auch Bicarbonate und Sesquicarbonate) und Sulfate.

Auch organische Builder gehören zu den verwendbaren Buildern. Dazu gehören Polycarboxylate, wie Ethercarboxylate, (cyclisch oder acyclisch), Hydroxypolycarboxylate, Copolymere aus Maleinsäureanhydrid und Ethylen oder Vinylmethylether, 1,3,5-Trihydroxybenzol-2,4,6-trisulfonsäure und Carboxymethoxybernsteinsäure, die alle in Form der Säure oder ihrer Alkali-, Ammonium- oder Organoammoniumsalze eingesetzt werden können. Alkyl-, Ammonium- oder Organoammoniumsalze der Polyessigsäure sind ebenso geeignet wie Salze der Zitronensäure oder Kombinationen von verschiedenen Buildern. Alkenylbernsteinsäuren und -salze sind besonders bevorzugte organische Builder. Monocarbonsäuresalze können ebenso entweder allein oder in Kombination mit einem der vorgenannten Builder in die Formulierungen eingearbeitet werden.

Die anorganischen (ohne Silikate) und/oder organischen Builder sind zu 0 bis 40 % enthalten.

e) Schmutzlösepolymere: Sämtliche zum Stand der Technik gehörenden Schmutzlösepolymere können als Ingredienzien in den erfindungsgemäßen Formulierungen eingesetzt werden. Als Bestandteil von Formulierungen tragen Schmutzlösepolymere zu einer leichteren Ablösung von Öl- und Fettschmutz bei, insbesondere bei Waschvorgängen und bei der Textilveredelung. Schmutzlösepolymere zeichnen sich dadurch aus, daß sie sowohl hydrophile wie auch hydrophobe Bauelemente besitzen. Die Wirkungsweise von Schmutzlösepolymeren beruht auf einer Modifizierung der Faseroberfläche von Polyester- bzw. Baumwoll/Polyestermischgeweben mit Hilfe des hydrophilierenden Polymers. Dabei bewirkt das hydrophile Segment des Schmutzlösepolymers eine leichtere Benetzung der Oberfläche, während das hydrophobe Segment als Ankergruppe fungiert.

Der Feuchtigkeitstransport (Wasserabsorption und Saugfähigkeit) wird bei den mit dem Schmutzlösepolymer behandelten hydrophoben Geweben wie Polyester oder Polyester/Baumwollmischgeweben erheblich verbessert. Außerdem verleihen sie den Stoffen antistatische und Gleiteigenschaften, wodurch die Handhabung dieser Fasern beim Schneiden und Nähen (Textilverarbeitung) erleichtert wird. Die Behandlung des Gewebes mit dem Schmutzlösepolymer ist als eine Art Imprägnierung zu verstehen, d.h. das Schmutzlösepolymer verbleibt für mehrere Waschcyclen auf der Faser.

Zur wichtigsten Gruppe von Schmutzlösepolymeren gehören Poly- bzw. Oligoester auf Basis Terephthalsäure/Polyoxyalkylenglykole/monomere Glykole. Schmutzlösepolymere dieser Gruppe werden schon seit mehreren Jahren vermarktet. Zu den wichtigsten Verkaufsprodukten zählen u.a. ZELCON (Du Pont), MILEASE T (ICI), ALKARIL QCF/QCJ (Alkaril Inc.) und REPEL-O-TEX (Rhone-Poulenc). Bevorzugt sind im Rahmen dieser Erfindung Schmutzlösepolymere, die sich durch folgende empirische Summenformel beschreiben lassen:

(CAP)ₓ(EG/PG)_{y}(T)_{z}(I)_{q}(DEG)ₛ(En)ₜ(CAR)ᵣ

Dabei repräsentiert **(CAP)** sog. "capping groups", die das Polymer am Ende verschließen. Der Endgruppenverschluß trägt zur Stabilisierung der Polymeren bei. Dabei steht **(CAP)** für eine Vielzahl von möglichen Endgruppen. Bevorzugte Endgruppen sind u.a. Sulfo-aroylgruppen, wie z.B. die Sulfobenzoyl-Gruppe, die in Form einer Umester g mit Sulfobenzoesäurealkylester eingeführt werden kann. Der Einbau von Endgruppen wirkt sich dabei zum einen regulierend auf das Molekulargewicht aus, andererseits führt er zur Stabilisierung der gewonnenen Polymeren. Neben Sulfoaroyl-Gruppen können auch ethoxylierte oder propoxylierte Hydroxyethan- und Hydroxypropansulfonate, so wie sie beispielsweise in WO 95/02029 und WO 95/02030 beschrieben sind, eingesetzt werden.

Bevorzugte Endgruppen sind auch Poly(oxyethylen)monoalkylether, in denen die Alkylgruppe 1 bis 30 C-Atome enthält und bei denen die Polyoxyethylengruppe aus 2-200 Oxyethyleneinheiten besteht. Endgruppen dieser Art werden z.B. in WO 92/17523 und DE 40 01 415 beschrieben. In EP 0 253 567 und EP 0 357 280 werden im besonderen solche endgruppenverschlossenen Polyester (capped polyesters) beschrieben, die zum einen durch nichtionische Gruppen wie z.B. C1-C4-Alkyl, C1-C4-Hydroxylalkyl, C1-C4-Acyl als auch durch ionische Succinatgruppen verschlossen werden. Grundsätzlich sind auch Mischungen verschiedener Endgruppen möglich, wobei x Werte von 0-2 annimmt.

Die Gruppe **(EG/PG)** steht für eine Oxyethylenoxy-, Oxypropylenoxy-Gruppe oder Mischungen davon, wobei y Zahlenwerte von 0 bis 80 annimmt. Als eine Variation der o.g. Polyester wird auch das Einbringen von verzweigten monomeren Glykolbausteinen beschrieben wie 1,2-Butylen- und 3-Methoxy-1,2-Propylenglykolen (EP 0 241 985).

Die Gruppe **(T)** steht u.a. für eine Terephthaloylgruppe, die im Polymer sozusagen als Ankergruppe zwischen Schmutzlösepolymer und Substrat (Faser) fungiert. Bei sämtlichen zum Stand der Technik gehörenden Schmutzlösepolymeren auf Oligo- bzw. Polyesterbasis ist die Gegenwart von Terephthaloyl-Gruppen essentiell für die Performance dieses Additivs. Neben Terephthaloylgruppen können jedoch auch aliphatische Analoga vertreten sein, wie z.B. Adipate, die durch Adipinsäure bzw. Adipinsäurediester eingebaut werden können. In DE 44 03 866 werden z.B. amphiphile Polyester beansprucht, die neben aromatischen auch aliphatische Dicarbonsäuren beinhalten und als Waschmitteladditiv bzw. Schmutzlösepolymer eingesetzt werden. Dabei nimmt z Werte von 1 bis 50 an.

Jedes **(I)** steht für eine interne anionische Gruppe mit q = 0-30. Der Einbau von anionischen Gruppen, in erster Linie Sulfoisophthaloyl-Gruppen in das Polymergerüst, stellt sich als sehr vorteilhaft für die Performance des Schmutzlösepolymers heraus. Die Sulfoisophthaloyl-Gruppen stabilisieren das Polymer und inhibieren den Übergang von der gewünschten amorphen Form zur schlechter löslichen kristallinen Form des Polymers. In US 4 427 557 und EP 0 066 944 werden solche anionischen Modifikationen der o.g. Polyester beschrieben, die als eine weitere Polymerisationskomponente das Natriumsalz der Sulfoisophthalsäure beinhalten. Die polymerisierten Polyethylenglykole (PEG) besitzen Molmassen von 200-1000 und ergeben nach ihrer Polymerisation mit Ethylenglykol und Terephthalsäure Polyester mit Molgewichten von 2 000-10 000.

**DEG** steht für eine Di(oxyethylen)oxy -Gruppe mit s = 0 - 80.

Jedes **(En)** steht für eine Poly(oxyalkylen)oxy-Gruppe, die aus 2 bis 100 Oxyalkylengruppen aufgebaut ist, wobei t = 0-25 ist und die Alkylgruppen 2-6 C-Atome enthalten. In den meisten Fällen handelt es sich bei den Poly(oxyalkylen)oxy-Gruppen um Poly(oxyethylen)oxy-Gruppen, wobei die Molekulargewichte erheblich variieren können. Neben Poly(oxyethylen)oxy-Gruppen können auch Poly(oxypropylen)oxy-Gruppen vertreten sein sowie alle denkbaren Mischungen. In DE 14 69 403 wird z.B. ein Verfahren zur oberflächenverändernden Behandlung von aus Polyestern abgeleiteten Artikeln beschrieben. Dabei sind die hergestellten Polyester aus ET-Einheiten aufgebaut mit ET:POET=2-6:1, wobei Polyethylenglykole mit Molgewichten von 1000-4000 eingesetzt werden. US 3 959 230 beansprucht ET/POET-Polyester mit ET:POET=25:75-35:65, wobei niedermolekulare Polyethylenglykole mit Molgewichten von 300-700 eingesetzt werden und die gewonnenen Polyester Molgewichte von 25 000-55 000 aufweisen.

Jedes **(CAR)** steht für eine Carbonylgruppe (C=O) einer Carbonateinheit, wobei r eine Zahl von 0 bis 80 darstellt. Es zeigt sich, daß durch Einbau von Carbonatgruppen in Form von Kohlensäureestern in das Polymergerüst die Performance der Schmutzlösepolymere weiterhin gesteigert werden kann. Einerseits lassen sich Polymere dieses Typs leichter dispergieren, zum anderen ist es möglich, mit Hilfe von Kohlensäureestern fließfähige, pumpbare Polymere zu erhalten, was hinsichtlich der meist üblichen Konfektionierung deutliche Vorteile bringt.
Die durch die obige empirische Summenformel beschriebenen Schmutzlösepolymere auf Poly- bzw. Oligoesterbasis besitzen Molekulargewichte zwischen 200 und 100 000. Bevorzugt sind meist Molekulargewichte im Bereich von 500 bis 25 000.

Neben dieser Klasse von Schmutzlösepolymeren können im Rahmen der Formulierungen auch Cellulose-Derivate eingesetzt werden. Solche Produkte sind kommerziell erhältlich z.B. als Hydroxyether der Cellulose unter der Produktbezeichnung METHOCEL (Dow). Bevorzugt sind Cellulose-Derivate mit C1-C4-Alkyl- und C4-Hydroxyalkylcellulosen (US 4 000 093). Als weitere Gruppe von Schmutzlösepolymeren können auch Poly(vinylester)-Verbindungen eingesetzt werden. Hierbei sind insbesondere Pfropfpolymerisate von Polyvinylacetat und Polyoxyethylenglykol bevorzugt. Produkte dieser Art sind marktgängig wie z.B. SOKALAN HP 22 (BASF).

Soweit Schmutzlösepolymere in den erfindungsgemäßen Formulierungen eingesetzt werden, beträgt der Gehalt 0,01 bis 10,0 Gew.-%. Bevorzugt ist ein Gehalt von 0,1 bis 5 Gew.-% bezogen auf die entsprechende Formulierung.

f) Chelatbildende Agentien: In den erfindungsgemäßen Formulierungen sind optional auch Eisen- und Manganionen unter Chelatbildung komplexierende Agentien enthalten, die zu der Gruppe der Aminocarboxylate, Aminophosphonate und polyfunktionalisierten Aromaten (z.B. Dihydroxybenzolsulfonsäurederivate) gehören. Auch Mischungen der verschiedenen Chelatisierungs-Agentien sind wirksam. Ein bevorzugtes bioabbaubares chelatbildendes Agenz ist Ethylendiamindisuccinat. Die vorgenannten Agentien werden in Anteilen von 0,1 bis 10 %, besonders bevorzugt von 0,1 bis 3,0 %, der Waschmittelformulierung eingesetzt.

g) Komponenten zur Entfernung von Ton- oder Lehmschmutz und Verhinderung der Wiederanschmutzung: Die erfindungsgemäßen Formulierungen können zu diesem Zweck alkoxylierte, bevorzugt ethoxylierte, Amine, unabhängig davon, ob es sich hier um mono-, oligo- oder polymere Amine handelt, enthalten. Für feste Formulierungen liegt die Einsatzmenge bei 0,01 bis 10 %, bei Flüssigformulierungen bei 0,01 bis 5 % der Gesamtformulierung. Andere Gruppen von Verbindungen, die diese Eigenschaften aufweisen, sind kationische Verbindungen (wie in EP-A-0 111 984), zwitterionische Polymere (wie in EP-A-0 112 592) oder Carboxymethylcellulose, die ebenfalls das Schmutztragevermögen einer Waschflottte zu steigern vermögen.

h) Polymere Dispersionshilfen (Cobuilder): Diese Additive werden in Mengen von 0,1 bis 7,0 % der erfindungsgemäßen Gesamtformulierung eingesetzt, wobei es sich um Polycarboxylate oder um Polyethylenglykole handelt, die sowohl die Wirkung des eingesetzten Builders verstärken als auch Inkrustierungen und Wiederanschmutzungen verhindern und bei der Ablösung von Partikelschmutz eine Rolle spielen. Die hier einsetzbaren Verbindungen werden durch Polymerisation oder Copolymerisation von geeigneten ungesättigten Carbonsäure- oder Carbonsäureanhydridmonomeren erhalten. Hier sind Polyacrylate aber auch Maleinsäureanhydrid/Acrylsäure-Copolymerisate bevorzugt. Die Molekulargewichte der ersteren bewegen sich in einem Bereich von 2 000 bis 10 000, bevorzugt 4 000 bis 7 000 und besonders bevorzugt im Bereich von 4 000 bis 5 000. Geeignete Copolymerisate weisen Molgewichte von 2 000 bis 100 000, bevorzugt 5 000 bis 75 000 und besonders bevorzugt 7 000 bis 65 000 auf. Verwendbare Polyethylenglykole weisen Molgewichte im Bereich 500 bis 100 000, besonders bevorzugt 1 500 bis 10 000 auf. Auch Polyasparagate und- glutamate können zusammen mit Zeolith-Buildern eingesetzt werden, wobei die verwendbaren Polyasparagate mittlere Molgewichte von ca. 10 000 aufweisen.

i) Optische Aufheller: Alle nach dem Stand der Technik bekannten optischen Aufheller sind in den erfindungsgemäßen Formulierungen einsetzbar. Sie werden zu 0,05 bis 1,2 %, bezogen auf die Gesamtformulierung, eingearbeitet. Einige nicht einschränkende Beispiele für geeignete Verbindungsgruppen seien im folgenden genannt: Stilbenderivate, Pyrazoline, Cumarin, Carbonsäuren, Methincyanine, Dibenzothiophen-5,5-dioxid, Azole, 5- und 6-gliedrige Heterocyclen.

j) Schauminhibitoren: Je nach genauer Zusammensetzung (d. h. Schäumvermögen der verwendeten Tenside) und Art des Schauminhibitors müssen 0 bis 5 % (bezogen auf Gesamtformulierung) davon eingesetzt werden. Monofettsäuresalze werden in einer Menge von 0 bis zu 5 %, bevorzugt 0,5 bis 3 % eingesetzt, Silicone werden in einer Menge bis zu 2 %, bevorzugt 0,01 bis 1 % und besonders bevorzugt von 0,25 bis 0,5 % eingesetzt. Zu den Verbindungen, die in den erfindungsgemäßen Formulierungen als Schauminhibitor eingesetzt werden können, gehören Monofettsäuren und ihre Salze mit C-Kettenlängen von 10 bis 24, bevorzugt 12 bis 18 Kohlenstoffatomen. Auch hochmolekulare nicht oberflächenaktive Verbindungen, wie Paraffine, Fettsäureester (z. B. Triglyceride), aliphatische Ketone, N-alkylierte Aminotriazine oder Di- bis Tetraalkyldiaminchlortriazine, Monostearylphosphate und Monostearylalkoholphosphatester können eingesetzt werden. Auch Mischungen von Siliconen und Silan-modifizierte Silikaten, i. a. mit Polyalkylenglykolen als Lösungsmittel, können als Schauminhibitoren eingesetzt werden.

k) Textilweichmacher: Verschiedene im Waschprozess verwendbare Textilweichmacher können hier verwendet werden, besonders jedoch Smectit-Tone sowie andere weichmachende Tone in Mengen zwischen 0,5 und 10 % (bezogen auf die Gesamtformulierung). Die vorgenannten Weichmacher können in Kombination mit anderen Weichmachern wie Aminen und den verbreiteten kationischen Weichmachern verwendet werden.

l) Tenside: Neben den anionischen Geminitensiden gemäß der Formel (I) können jeweils Kombinationen oder einzelne der im folgenden genannten Tenside mit den Geminitensiden in den erfindungsgemäßen Formulierungen kombiniert werden. Hierbei werden 0,1 bis 70 % dieser Tenside eingesetzt:

Ohne die Formulierungen darauf einzuschränken, seien als Beispiele für nichtionische grenzflächenaktive Substanzen Fettsäureglyceride, Fettsäurepolyglyceride, Fettsäureester, Alkoxylate von C₆-C₃₀-Alkoholen, alkoxylierte Fettsäureglyceride, Polyoxyethylenoxypropylenglykolfettsäureester, Polyoxyethylensorbitanfettsäureester, Polyoxyethylen-Rhizinusöl- oder gehärtete Rhizinusölderivate, Polyoxyethylenlanolinderivate, Polyoxyethylenfettsäureamide, Polyoxyethylenalkylamine, Derivate von Alkanolaminen, Derivate von Alkylaminen, Alkylaminoxide, Derivate von Eiweißhydrolysaten, Hydroxymischether, Alkylpolyglycoside und Alkylglucamide (z. B. N-Methylalkylglucamide) sowie nichtionische Geminitenside bzw. verbrückte nichtionische Tenside (wie in WO 95/19951 (Polyhydroxyaminverbindungen), WO 95/19953, WO 95/19954 und WO 95/19955 sowie WO 95/20026 beschrieben) genannt.

Als Beispiele für anionische grenzflächenaktive Substanzen, die für Kombinationen eingesetzt werden können, seien Seifen, Ethercarbonsäuren und deren Salze, Alkylsulfonate, α-Olefinsulfonate, α-Sulfofettsäurederivate (einschließlich der in W093/25646 beschriebenen), Sulfonate höherer Fettsäureester, höhere Alkoholsulfate (primär und sekundär), Alkoholethersulfate, Hydroxymischethersulfate, Sulfate von alkoxylierten Carbonsäurealkanolamiden, Salze von Phosphatestern, Tauride, Isethionate, lineare Alkylbenzolsulfonate, verbrückte Alkylbenzolsulfonate (wie DOWFAX-Typen der Firma Dow), Alkylarylsulfonate, Sulfate der Polyoxyethylenfettsäureamide und Derivate von Acylaminosäuren, Alkylethercarbonsäuren, Alkyl- und Dialkylsulfosuccinate, Alkenylsulfosuccinate, Alkyl- oder Alkenylsarcosinate und sulfatierte Glycerinalkylether genannt.

Als Beispiele für kationische gängige grenzflächenaktive Substanzen, die für Kombinationen eingesetzt werden können, seien Alkyltrimethylammoniumsalze, Dialkyldimethylammoniumsalze, Alkyldimethylbenzylammoniumsalze, Imidazoliniumderivate, Alkylpyridiniumsalze, quaternierte Fettsäureester von Alkanolaminen, Alkylisochinoliniumsalze, Benzethoniumchloride und kationische Acylaminosäurederivate genannt.

Als Beispiele für ampholytische und betainische grenzflächenaktive Substanzen, die für Kombinationen eingesetzt werden können, seien Carbobetaine, wie z. B. Kokosacylamidopropyldimethylbetain, Acylamidopentandiethylbetain, Di-methylammoniohexanoat -acylamidopropan-(oder -ethan-)dimethyl (oder- diethyl-)betain - alle mit C-Kettenlängen zwischen 10 und 18 , Sulfobetaine, Imidazolinderivate, Sojaöllipide und Lecithin genannt. Die oben erwähnten Amin-N-oxide können auch in polymerer Form vorliegen, wobei ein Verhältnis Amin- zu Amin-N-oxid von 10 : 1 bis 1 : 1 000 000 vorliegen muß. Die mittlere Molmasse beträgt 500 bis 1 000 000, besonders bevorzugt jedoch 5 000 bis 100 000.

m) Weitere Komponenten: Weitere Komponenten können in die Wasch-, Reinigungs- oder Körperpflegemittelformulierungen eingebaut werden: Weitere Träger, Hydrotropica, Prozesshilfsmittel, Farbstoffe oder Pigmente, Parfums, Lösungsmittel für Flüssigformulierungen (besonders bevorzugt sind Alkohole mit 1 bis 6 Kohlenstoffatomen und 1 bis 6 Hydroxygruppen), feste Füller für Stückseifenformulierungen, Perlglanzmittel, z. B. Distearoylglyceride, Konservierungsmittel, Pufferungssysteme und so weiter. Sollte ein höheres Schäumvermögen der Formulierung, wie z. B. in einigen Körperpflegemitteln, erforderlich sein, so kann dieses z. B. durch den Zusatz von C₁₀-C₁₆-Alkanolamiden (in Konzentrationen von 1 bis 10 % der Gesamtformulierung) erhöht werden. Auch weitere wasserlösliche Magnesiumsalze können

zur Erhöhung des Schäumvermögens und der Fettlösekraft in Mengen von 0,1 bis 2 % zugesetzt werden.
Wenn notwendig, können einige der obengenannten Tensidkomponenten auch durch Adsorption auf poröse hydrophobe Substanzen stabilisiert und mit einer weiteren hydrophoben Schicht versiegelt in die Formulierung eingearbeitet werden.

Verwendung: Die erfindungsgemäßen Formulierungen können sehr vielseitig verwendet werden wie z. B. als Hautreiniger, Badezusatz, Duschbad, Gebißreiniger, Haarwasch- und -pflegemittel, Rasierseife, Pre-shaving, Desinfektionsmittel, Dispergiermittel, Universalwaschmittel und Feinwaschmittel, sowohl in flüssiger Form als auch als Pulver oder Granulat, für Textilien, Hand- und Maschinengeschirrspülmittel, Haushaltsreiniger und Industriereiniger.

### Beispiele

Die folgenden Beispiele, die keinen die Ansprüche einschränkenden Charakter haben sollen, zeigen die positiven anwendungstechnischen Eigenschaften der Geminitenside gemäß Formel (I), die sie für den Einsatz in den o. g. Verwendungszwecken prädestinieren.

### A) Reinigung von harten Oberflächen

Zur Bestimmung der Reinigungswirkung an harten Oberflächen wurden je 7 Trägerstreifen aus Hart-PVC (455 x 39 x 2,5 mm) eng nebeneinander gelegt und auf einer Seite der Trägerstreifen eine weiße PVC-Folie (400 x 400 mm) blasenfrei aufgelegt. Anschließend wurden Schablonen aus PVC (300 x 350 x 3 mm) mit 260 x 280 mm-Ausschnitt so auf die Folie plaziert, daß der Ausschnitt über der Mitte der Folie lag. Innerhalb des Ausschnittes wurden 2 g eines Testschmutzes (7 Gew.-% Spezialschwarz 4, Fa. Degussa, 17 Gew.-% Myritol 318, Fa. Henkel, 40 Gew.-% Prozeßöl 310, Fa. Esso, 36 Gew.-% Exxon DSP 80/100, Fa. Exxon) mittels eines Flachpinsels auf der Folie verteilt. Die Trägerstreifen wurden mit einem Messer getrennt. Je ein Teststreifen wurde dann in die Metallführungsschiene eines Dardner-Waschbarkeits- und Scheuerprüfgerätes (Modell M-105-A, Fa. Erichsen) gelegt und mit einem feuchten Schwamm, der in einem Schlitten plaziert war, mit 16 ml der zu untersuchenden tensidhaltigen Formulierung (Tensidgehalt: 1 Gew.-%) gereinigt. Dies geschah durch 10 maliges Hin- und Herbewegen des Schlittens. Nach dem Trocknen der Teststreifen wurde die PVC-Folie vom Trägerstreifen gelöst und mit der gereinigten Seite nach oben auf weiße Pappe geklebt. Die Reinigungsleistung der jeweiligen tensidhaltigen Formulierung wurde spektralphotometrisch mit Hilfe eines Spektralphotometers "datacolor 3890" bei einer Wellenlänge von 560 nm relativ zu einem Weißstandard (= 100 %) und einem angeschmutzten Standard (= 0 %) bestimmt.

### (Vergleichsbeispiel):

Als Standardformulierung wurde die Reinigungsleistung eines marktgängigen Allzweckreinigers mit 65 % bestimmt.

### Erfindungsgemäße Beispiele:

Die Reinigungsleistungen der erfindungsgemäßen anionischen Geminitenside wurden anhand der folgenden Rezeptur bestimmt:

| | |
|---|---|
| anionisches Geminitensid | 3,0 Gew.-% |
| Marlipal 0 13/80* | 1,0 Gew.-% |
| Kokosfettsäure-Na-salz | 0,25 Gew.-% |

| | |
|---|---|
| *) C₁₃-Alkohol mit 8 Ethylenoxideinheiten (Fa. Hüls) | |

Folgende anionische Geminitenside wurden untersucht:
1: Gemäß Formel (I), mit R¹ = R³ = C₇-C₉-Alkyl, R² = C₆H₁₂, X = Y = (C₂H₄O)_{a,b},SO₃Na, a + b = 8, z = 1;
2: Gemäß Formel (I), mit R¹ = = R³ = C₇-C₉-Alkyl, R² = C₆H₁₂, X = Y = (C₂H₄O)_{a,b},SO₃Na, a + b = 20, z = 1;
3. Gemäß Formel (I), mit R¹ = R³ = C₇-C₉-Alkyl, R² = C₂H₄, X = Y = (C₂H₄O)_{a,b},SO₃Na, a + b = 16, z = 1;

### Beispiel 1:

Die Reinigungsleistung der Verbindung 1 enthaltenden Rezeptur wurde mit 72 % bestimmt und ist somit höher als die im Vergleichsbeispiel genannte.

### Beispiel 2:

Die Reinigungsleistung der Verbindung 2 enthaltenden Rezeptur wurde mit 74 % bestimmt und ist damit höher als die im Vergleichsbeispiel genannte.

### Beispiel 3:

Die Reinigungsleistung der Verbindung 3 enthaltenden Rezeptur wurde mit 66 % bestimmt und ist damit höher als die im Vergleichsbeispiel genannte.

### B) Waschvermögen von Textilien

Zur Bestimmung des Waschvermögens von Textilien wurde die Waschtrommel einer marktgängigen Haushaltswaschmaschine mit 4 kg (für 95 °C und 60 °C-Wäsche) bzw. mit 1 kg (für 30 °C-Wäsche) sauberem Baumwoll-Ballastgewebe von der Fa. Carl Laarmann (Art.-Nr. 5005 HE) beschickt. Weiterhin wurden mit Hautfett-Pigment-Anschmutzung behandelte Prüfläppchen der WFK-Testgewebe GmbH mit der testverschmutzten Seite nach oben auf ein weißes Baumwollhandtuch (Fa. Carl Laarmann, Art.-Nr.: HL DIN G 25) aufgenäht. Das so präparierte Baumwollhandtuch wurde in die Trommel der Haushaltswaschmachine zum Ballastgewebe gegeben. Folgende WFK-Testgewebe wurden verwendet:

| | |
|---|---|
| Baumwolle (BW). | 10 D |
| Baumwoll-Polyester.Mischgewebe (MG) | 20 D |
| Polyester (PE) | 30 D |

Die Waschvermögen der erfindungsgemäßen anionischen Geminitenside wurden anhand der folgenden Rezeptur eines flüssigen Vollwaschmittels bestimmt:

| | |
|---|---|
| Anionisches Geminitensid | 13,0 Gew.-% |
| MARLIPAL 24/70*¹ | 15,5 Gew.-% |
| KOH, 50 %ig | 3,9 Gew.-% |
| Kokosfettsäure | 10,0 Gew.-% |
| Triethanolamin | 5,0 Gew.-% |
| 1,2-Propylenglykol | 3,0 Gew.-% |
| Ethanol | 8,0 Gew.-% |
| Dequest 2060 S*² | 1,5 Gew.-% |
| Rest zu 100 Gew.-Wasser | |

| | |
|---|---|
| *1 C_{12/14} -Alkohol mit 7 Ethylenoxideinheiten (Fa. Hüls) | |
| *2 Diethylentriamin-penta-(meth.phosph.säure) (Fa. Monsanto) | |

Die Einsatzmenge des flüssigen Vollwaschmittels betrug jeweils 25 g pro Waschgang. Nach erfolgter Wäsche wurden die WFK-Testgewebe getrocknet. Die Bestimmung des Waschvermögens erfolgte spektralphotometrisch durch Ermittlung der Remission mit Hilfe des Spektralphotometers datacolor 3890 (Weißstandard: 100 % Remission, testangeschmutztes Gewebe: 0 % Remission).

Folgende anionische Geminitenside wurde untersucht:
4: Gemäß Formel (I), mit
   R¹=R³ =C₇ -C₉ -Alkyl, R² =C₄H₈, X=Y=(C₂H₄O) _{a,b}(SO₃Na)_{1,16}, a+b=17,3, z=1;
5: Gemäß Formel (I), mit
   R¹=R³=C₁₁ -C₁₃ -Alkyl, R²=C₆H₁₂, X=Y=(C₂H₄O)_{a,b}(SO₃Na)_{1,65}, a+b=17,9, z=1;

Um die Waschvermögen besser beurteilen zu können, wurde anstelle der Geminitenside im Vergleichsbeispiel das üblicherweise verwendete MARLON PS (sek.-Alkansulfonat-Na-salz), Hüls AG) in der gleichen Konzentration in die o. g. Rezeptur eingesetzt.
Gefundene Remissionswerte in %:

| In der Rezeptur verwendetes Tensid | PE 30 °C | MG 30 °C | MG 60 °C | BW 60 °C | BW 95 °C |
|---|---|---|---|---|---|
| sek. Alkansulfonat-Na-salz (nicht erfindungsgemäß) | 49 | 44 | 30 | 24 | 25 |
| 4 | 58 | 44 | 30 | 30 | 31 |
| 5 | 52 | 48 | 33 | 30 | 40 |

Es zeigt sich, daß 4 und 5 gleiche bzw. wesentlich bessere Remissionswerte bewirken als das sek.-Alkansulfonat-Na-Salz.

## Patentansprüche

1. Wasch-, Reinigungs- und Körperpflegemittel unter Verwendung von mindestens 0,1 Gew.-% anionischen Geminitensiden der allgemeinen Formel I 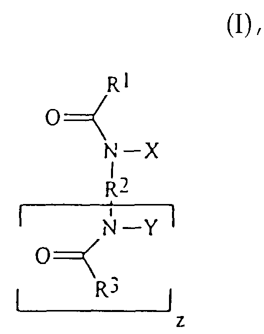 wobei in der Formel I R¹ und R³ unabhängig voneinander einen Kohlenwasserstoffrest mit 1 bis 22 Kohlenstoffatomen,
R² einen Spacer aus einer unverzweigten oder verzweigten Kette mit 2-100 Kohlenstoffatomen, die 0-20 Sauerstoffatome, 0-20 Stickstoffatome, 0-4 Schwefelatome und/oder 0-3 Phosphoratome enthält und die 0-20 funktionelle Seitengruppen aufweist und der 0 bis 20 Alkoxygruppen enthält,
und X oder Y einen Substituenten der Formel II
-(CH₂H₄O)_{α}(C₃H₆O)_{β}H (II)
mit α = 0 bis 50,
β = 0 bis 60
und α + β = 1 bis 100,
und X oder Y einen Substituenten der Formel III
oder
X und Y unabhängig voneinander Substituenten der Formel III
-(C₂H₄O)_{γ}(C₃H₆O)_{δ}FR (III),
wobei FR für einen funktionellen Rest -CH₂-COOM, -SO₃M, -P(O)(OM)₂, -O-C(O)-(C₂H₃(SO₃M)-CO₂M' oder -C₂H₄-SO₃M mit M, M'= Alkali-, Ammonium-, Alkanolammonium- oder 1/2 Erdalkaliion steht,
mit γ = 0 bis 20,
δ = 0 bis 20
und γ + δ = 1 bis 40,
und wobei die Alkoxideinheiten bei den Verbindungen gemäß den Formeln II und III statistisch oder blockweise eingebunden sind und die Reihenfolge beliebig ist, bedeutet,
und wobei der Substitutionsgrad Z = 1 bis 10 beträgt.

2. Mittel nach Anspruch 1 unter Verwendung von mindestens 0,1 Gew.-% anionischen Geminitensiden der allgemeinen Formel I,
dadurch gekennzeichnet,
daß die Kohlenwasserstoffreste R¹ und R³ unabhängig voneinander für einen unverzweigten oder verzweigten, gesättigten oder ungesättigten Kohlenwasserstoffrest mit 1 bis 22 Kohlenstoffatomen stehen, R² einen Spacer, bestehend aus einer unverzweigten oder verzweigten Kette mit 2 bis 100 Kohlenstoffatomen, die 0 bis 20 Sauerstoff-, 0 bis 20 Stickstoff-, 0 bis 4 Schwefel- und/oder 0 bis 3 Phosphoratome enthält und die 0 bis 20 Hydroxyl-, Carbonyl-, Carboxyl-, Amino- und/oder Acylaminogruppen enthält, bedeutet

3. Mittel nach Anspruch 1 oder 2 unter Verwendung von mindestens 0,1 Gew.-% anionischen Geminitensiden der allgemeinen Formel I,
dadurch gekennzeichnet,
daß die Geminitenside bei gleicher Grundstruktur eine Mischung von Homologen mit verschieden langen lipophilen Kohlenwasserstoffresten sind.

4. Mittel gemäß einem der vorhergehenden Ansprüche unter Verwendung von mindestens 0,1 Gew.-% anionischen Geminitensiden der allgemeinen Formel I,
dadurch gekennzeichnet,
daß die Geminitenside bei gleicher Grundstruktur eine Mischung von Homologen mit verschieden langen Spacern R² sind.

5. Mittel gemäß einem der vorhergehenden Ansprüche unter Verwendung von mindestens 0,1 Gew.-% anionischen Geminitensiden der allgemeinen Formel I,
dadurch gekennzeichnet,
daß die Geminitenside bei gleicher Grundstruktur eine Mischung von Homologen mit verschieden langen Alkoxyketten im Spacer R² und/oder in den funktionellen Gruppen X und Y sind.

6. Wasch-, Reinigungs- und Körperpflegemittel gemäß einem der vorhergehenden Ansprüche unter Verwendung von mindestens 0,1 Gew.-% anionischen Geminitensiden der allgemeinen Formel I,
dadurch gekennzeichnet,
daß weitere anionische, nichtionische, kationische, betainische und/oder amphotere grenzflächenaktive Verbindungen enthalten sind.

7. Mittel gemäß Anspruch 6 unter Verwendung von mindestens 0,1 Gew.-% anionischen Geminitensiden der allgemeinen Formel I,
dadurch gekennzeichnet,
daß als nichtionische grenzflächenaktive Verbindungen Fettsäureglyceride, Fettsäurepolyglyceride, Fettsäureester, Alkoxylate von C₆ - C₃₀ - Alkoholen, alkoxylierte Fettsäureglyceride, Polyoxyethylenoxypropylenglykolfettsäureester, Polyoxyethylensorbitanfettsäureester, Polyoxyethylen-Rhizinusol- oder gehärtete Rhizinusölderivate, Polyoxyethylenlanolinderivate, Polyoxyethylenfettsäureamide, Polyoxyethylenalkylamine, Derivate von Alkanolaminen, Derivate von Alkylaminen Alkylaminoxide. Derivate von Eiweißhydrolysaten, Hydroxymischether, Alkylpolyglycoside, Alkylglucamide, nichtionische Geminitenside und nichtionische verbruckte Tenside und deren Mischungen eingesetzt werden.

8. Mittel gemäß Anspruch 6 unter Verwendung von mindestens 0,1 Gew -% anionischen Geminitensiden der allgemeinen Formel I,
dadurch gekennzeichnet,
daß als anionische grenzflächenaktive Verbindungen Seifen, Ethercarbonsäuren und deren Salze, Alkylsulfonate, α-Olefinsulfonate, α-Sulfofettsaurederivate, Sulfonate höherer Fettsäureester, höhere primäre und sekundäre Alkoholsulfate, Alkoholethersulfate, Hydroxymischethersulfate, Sulfate von alkoxylierten Carbonsäurealkanolamiden, Salze von Phosphatestern, Tauride, Isethionate, lineare Alkylbenzolsulfonate, verbruckte Alkylbenzolsulfonate, Alkylarylsulfonate, Sulfate der Polyoxyethylenfettsäureamide und Derivate von Acylaminosäuren, Alkylethercarbonsäuren, Alkyl- und Dialkylsulfosuccinate, Alkyl- oder Alkenylsarcosinate und sulfatierte Glycerinalkylether und deren Mischungen eingesetzt werden.

9. Mittel gemäß Anspruch 6 unter Verwendung von mindestens 0,1 Gew.-% anionischen Geminitensiden der allgemeinen Formel I,
dadurch gekennzeichnet,
daß als kationische grenzflächenaktive Verbindungen Alkyltrimethylammoniumsalze, Dialkyldimethylammoniumsalze, Alkyldimethylbenzylammoniumsalze, Imidazoliniumderivate, Alkylpyridiniumsalze, quaternierte Fettsäureester von Alkanolaminen, Alkylisochinoliniumsalze, Benzethoniumchloride und kationische Acylaminosäurederivate eingesetzt werden

10. Mittel gemäß Anspruch 6 unter Verwendung von mindestens 0,1 Gew.-% anionischen Geminitensiden der allgemeinen Formel I,
dadurch gekennzeichnet,
daß als grenzflächenaktive Betaine und Ampholyte Kokosacylamidopropylbetain, Acylamidopentandiethylbetain, Dimethylammoniohexanoat-acylamidopropan-(oder -ethan-)dimethyl(oder -diethyl-)betain mit C-Kettenlängen von 10 bis 18 C-Atomen, Sulfobetaine, Imidazolinderivate, Sojaollipide und Lecithin und deren Mischungen eingesetzt werden

11. Reinigungsmittel für Textilien unter Verwendung von mindestens 0,1 Gew.-% anionischen Geminitensiden der allgemeinen Formel I gemäß einem der vorhergehenden Ansprüche

12. Reinigungsmittel für harte Oberflächen unter Verwendung von mindestens 0,1 Gew.-% anionischen Geminitensiden der allgemeinen Formel I gemäß einem der vorhergehenden Anspruche

13. Manuelles Spülmittel unter Verwendung von mindestens 0,1 Gew.-% anionischen Geminitensiden der allgemeinen Formel I gemäß einem der vorhergehenden Ansprüche.

14. Reinigungsmittel für Haut und Haar unter Verwendung von mindestens 0,1 Gew.-% anionischen Geminitensiden der allgemeinen Formel I gemäß einem der vorhergehenden Ansprüche.

15. Körperpflegemittel unter Verwendung von mindestens 0,1 Gew.-% anionischen Geminitensiden der allgemeinen Formel I gemäß einem der vorhergehenden Ansprüche.

## Claims

1. Detergents, cleansers, and body care preparations utilizing at least 0.1 % by weight of anionic gemini surfactants of the general formula I 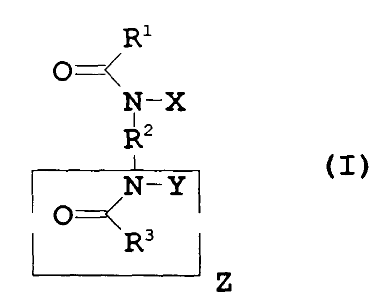 where in the formula I R¹ and R³, independently of one another, are a hydrocarbon radical having 1 to 22 carbon atoms,
R² is a spacer of an unbranched or branched chain having from 2 to 100 carbon atoms, which contains 0 to 20 oxygen atoms, 0 to 20 nitrogen atoms, 0 to 4 sulfur atoms, and/or 0 to 3 phosphorus atoms, and which comprises from 0 to 20 functional side groups, and which contains 0 to 20 alkoxy groups,
and X or Y is a substituent of the formula II
-(C₂H₄O)_{α}(C₃H₆O)_{β}H (II)
where α = 0 to 50,
β = 0 to 60,
and α + β = 1 to 100,
and X or Y is a substituent of the formula III or
X and Y, independently of one another, are substituents of the formula III
-(C₂H₄O)_{γ}(C₃H₆O)_{δ}FR (III)
wherein FR is a functional radical -CH₂-COOM, -SO₃M, -P(O)(OM)₂, -O-C(O)-C₂H₃(SO₃M)-CO₂M', or -C₂H₄-SO₃M,
where M, M' = an alkali, ammonium, alkanolammonium, or ½ alkaline earth metal ion,
where y = 0 to 20,
δ = 0 to 20,
and γ + δ = 1 to 40,
and where the alkoxide units in the compounds of the formulae II and III are incorporated randomly or in blocks and the sequence is arbitrary, and where the degree of substitution Z is from 1 to 10.

2. Agent according to claim 1 utilizing at least 0.1 % by weight of anionic gemini surfactants of the general formula I,
**characterized in that** the hydrocarbon radicals R¹ and R³, independently of one another, are an unbranched or branched, saturated or unsaturated hydrocarbon radical having 1 to 22 carbon atoms, R² is a spacer consisting of an unbranched or branched chain having 2 to 100 carbon atoms, which contains 0 to 20 oxygen atoms, 0 to 20 nitrogen atoms, 0 to 4 sulfur atoms, and/or 0 to 3 phosphorus atoms and which contains from 0 to 20 hydroxyl, carbonyl, carboxyl, amino, and/or acylamino groups.

3. Agent according to claim 1 or 2 utilizing at least 0.1 % by weight of anionic gemini surfactants of the general formula I,
**characterized in that** the gemini surfactants, with the same basic structure, are a mixture of homologues having lipophilic hydrocarbon radicals which differ in length.

4. Agent according to any one of the preceding claims utilizing at least 0.1 by weight of anionic gemini surfactants of the general formula I,
**characterized in that** the gemini surfactants, with the same basic structure, are a mixture of homologues with R² spacers which differ in length.

5. Agent according to any one of the preceding claims utilizing at least 0.1 % by weight of anionic gemini surfactants of the general formula I,
**characterized in that** the gemini surfactants, with the same basic structure, are a mixture of homologues with alkoxy chains which differ in length in the spacer R² and/or in the functional groups X and Y.

6. Detergents, cleansers, and body care preparations according to any one of the preceding claims utilizing at least 0.1 % by weight of anionic gemini surfactants of the general formula I,
**characterized in that** further anionic, nonionic, cationic, betaine and/or amphoteric surface-active compounds are present.

7. Agent according to claim 6 utilizing at least 0.1 % by weight of anionic gemini surfactants of the general formula I,
**characterized in that** the nonionic surface-active compounds employed are fatty acid glycerides, fatty acid polyglycerides, fatty acid esters, alkoxylates of C₆-C₃₀ alcohols, alkoxylated fatty acid glycerides, polyoxyethylene oxypropylene glycol fatty acid esters, polyoxyethylene sorbitan fatty acid esters, polyoxyethylene castor oil or dehydrated castor oil derivatives, polyoxyethylene lanoline derivatives, polyoxyethylene fatty acid amides, polyoxyethylene alkylamines, derivatives of alkanolamines, derivatives of alkylamines, alkylamine oxides, derivatives of protein hydrolysates, hydroxy mixed ethers, alkylpolyglycosides, alkylglucamides, nonionic gemini surfactants and nonionic bridged surfactants and mixtures thereof.

8. Agent according to claim 6 utilizing at least 0.1 % by weight of anionic gemini surfactants of the general formula I,
**characterized in that** the anionic surface-active compounds employed are soaps, ethercarboxylic acids and their salts, alkylsulfonates, α-olefin sulfonates, α-sulfo-fatty acid derivatives, sulfonates of higher fatty acid esters, higher primary and secondary alcohol sulfates, alcohol ether sulfates, hydroxy mixed ether sulfates, sulfates of alkoxylated carboxylic acid alkanolamides, salts of phosphate esters, taurides, isethionates, linear alkyl benzene sulfonates, bridged alkyl benzene sulfonates, alkylaryl sulfonates, sulfates of polyoxyethylene fatty acid amides and derivatives of acylamino acids, alkyl ether carboxylic acids, alkyl and dialkyl sulfosuccinates, alkyl or alkenyl sarcosinates and sulfated glycerol alkyl ethers and mixtures thereof.

9. Agent according to claim 6 utilizing at least 0.1 % by weight of anionic gemini surfactants of the general formula I,
**characterized in that** the cationic surface-active compounds employed are alkyltrimethyl ammonium salts, dialkyldimethyl ammonium salts, alkyl dimethylbenzyl ammonium salts, imidazolinium derivatives, alkylpyridinium salts, quaternized fatty acid esters of alkanolamines, alkylisoquinolinium salts, benzethonium chlorides, and cationic acylamino acid derivatives.

10. Agent according to claim 6 utilizing at least 0.1 % by weight of anionic gemini surfactants of the general formula I,
**characterized in that** the surface-active betaines and ampholytes employed are cocoacylamidopropylbetaine, acylamidopentane diethylbetaine, dimethylammoniohexanoate-acylamidopropane-(or -ethane-)dimethyl(or -diethyl-)betaine with carbon chain lengths from 10 to 18 carbon atoms, sulfobetaines, imidazoline derivatives, soya oil lipids and lecithin and mixtures thereof.

11. Textile cleansers utilizing at least 0.1 % by weight of anionic gemini surfactants of the general formula I according to any one of the preceding claims.

12. Cleansers for hard surfaces utilizing at least 0.1 % by weight of anionic gemini surfactants of the general formula I according to any one of the preceding claims.

13. Manual rinsing agents utilizing at least 0.1 % by weight of anionic gemini surfactants of the general formula I according to any one of the preceding claims.

14. Cleanser for skin and hair utilizing at least 0.1 % by weight of anionic gemini surfactants of the general formula I according to any one of the preceding claims.

15. Body care preparations utilizing at least 0.1 % by weight of anionic gemini surfactants of the general formula I according to any one of the preceding claims.

## Revendications

1. Agent de lavage, de nettoyage et de soins corporels avec utilisation d'au moins 0,1% en poids d'agents gem-tensioactifs anioniques de formule générale I : 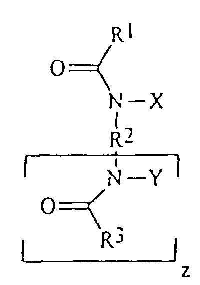 dans la formule I, R¹ et R³ signifiant, indépendamment l'un de l'autre, un radical hydrocarboné ayant 1 à 22 atomes de carbone,
R² signifiant un espaceur constitué d'une chaîne non ramifiée ou ramifiée avec 2-100 atomes de carbone qui contient 0-20 atomes d'oxygène, 0-20 atomes d'azote, 0-4 atomes de soufre et/ou 0-3 atomes de phosphore et présente 0-20 groupements latéraux fonctionnels et qui contient 0 à 20 groupements alcoxy,
et X ou Y signifiant un substituant de formule II :
-(C₂H₄O_{α} (C₃H₆O)_{β}H (II)
avec α = 0 à 50,
β = 0 à 60, et
α + β = 1 à 100,
et X ou Y signifiant un substituant de formule III : ou
X et Y signifiant, indépendamment l'un de l'autre, des substituants de formule III :
-(C₂H₄O)_{γ}(C₃H₆O)_{δ}FR (III)
où FR est un radical fonctionnel -CH₂-COOM, -SO₃M, -P(O)(OM)₂, -O-C(O)-C₂H₃(SO₃M)-CO₂M' ou -C₂H₄-SO₃M avec M,M' = ion d'alcali, d'ammonium, d'alcanolammonium ou ½ ion d'alcalino-terreux,
avec γ = 0 à 20,
δ = 0 à 20 et
γ + δ = 1 à 40,
où les unités alcoxyde sont intégrées de manière statistique ou en bloc aux composés de formules II et III et la séquence est quelconque, et
le degré de substitution Z s'élève à 1 jusqu'à 10.

2. Agent selon la revendication 1, avec utilisation d'au moins 0,1% en poids d'agents gem-tensioactifs anioniques de formule générale I, caractérisé en ce que les radicaux hydrocarbonés R¹ et R² sont, indépendamment l'un de l'autre, un radical hydrocarboné non ramifié ou ramifié, saturé ou insaturé, ayant 1 à 22 atomes de carbone, R² signifie un espaceur constitué d'une chaîne non ramifiée ou ramifiée ayant 2 à 100 atomes de carbone, qui contient 0 à 20 atomes d'oxygène, 0 à 20 atomes d'azote, 0 à 4 atomes de soufre et/ou 0 à 3 atomes de phosphore et qui contient également 0 à 20 groupements hydroxyle, carbonyle, carboxyle, amino et/ou acylamino.

3. Agent selon la revendication 1 ou 2, avec utilisation d'au moins 0,1 % en poids d'agents gem-tensioactifs anioniques de formule générale I, caractérisé en ce que les agents gem-tensioactifs sont, à structure de base égale, un mélange d'homologues avec des radicaux hydrocarbonés lipophiles de diverses longueurs.

4. Agent selon l'une quelconque des revendications précédentes, avec utilisation d'au moins 0,1% en poids d'agents gem-tensioactifs anioniques de formule générale I, caractérisé en ce que les agents gem-tensioactifs sont, à structure de base égale, un mélange d'homologues avec des espaceurs R² de diverses longueurs.

5. Agent selon l'une quelconque des revendications précédentes, avec utilisation d'au moins 0,1% en poids d'agents gem-tensioactifs anioniques de formule générale I, caractérisé en ce que les agents gem-tensioactifs sont, à structure de base égale, un mélange d'homologues avec des chaînes alcoxy de diverses longueurs dans l'espaceur R² et/ou dans les groupements fonctionnels X et Y.

6. Agent de lavage, de nettoyage et de soins corporels selon l'une quelconque des revendications précédentes, avec utilisation d'au moins 0,1% en poids d'agents gem-tensioactifs anioniques de formule générale I, caractérisé en ce qu'il contient d'autres composés tensioactifs anioniques, non ioniques, cationiques, bétaïniques et/ou amphotères.

7. Agent selon la revendication 6, avec utilisation d'au moins 0,1% d'agents gem-tensioactifs anioniques de formule générale I, caractérisé en ce qu'on utilise comme composés tensioactifs non ioniques des glycérides d'acides gras, des polyglycérides d'acides gras, des esters d'acides gras, des alcoxylates d'alcools en C₆-C₃₀, des glycérides d'acides gras alcoxylés, des esters d'acides gras et de polyoxyéthylèneoxypropylèneglycol, des esters d'acides gras et de polyoxyéthylènesorbitane, des dérivés de polyoxyéthylène et d'huile de ricin ou d'huile de ricin durcie, des dérivés de polyoxyéthylène et de lanoline, des amides de polyoxyéthylène et d'acides gras, des alkylamines de polyoxyéthylène, des dérivés d'alcanolamines, des dérivés d'alkylamines, des oxydes d'alkylamines, des dérivés d'hydrolysats protéiques, des hydroxyéthers mixtes, des alkylpolyglycosides, des alkylglucamides, des agents gem-tensioactifs non ioniques et des agents tensioactifs pontés non ioniques et leurs mélanges.

8. Agent selon la revendication 6, avec utilisation d'au moins 0,1% en poids d'agents gem-tensioactifs anioniques de formule générale I, caractérisé en ce qu'on utilise comme composés tensioactifs anioniques des savons, des acides éthercarboxyliques et leurs sels, des alkylsulfonates, des α-oléfinesulfonates, des odérivés d'acides gras α-sulfoniques, des sulfonates d'esters d'acides gras supérieurs, des sulfates d'alcools primaires et secondaires supérieurs, des éthersulfates d'alcools, des hydroxy-éthersulfates mixtes, des sulfates d'alcanolamides d'acides carboxyliques alcoxylés, des sels d'esters phosphates, des taurides, des iséthionates, des alkylbenzènesulfonates linéaires, des alkylbenzène sulfonates pontés, des sulfonates d'alkylaryle, des sulfates d'amides d'acides gras polyoxyéthyléniques et des dérivés d'acides acylaminés, des acides alkyléthercarboxyliques, des succinates d'alkyle et de dialkyle, des sarcosinates d'alkyle ou d'alcényle et des éthers alkyliques de glycérol sulfatés et leurs mélanges.

9. Agent selon la revendication 6, avec utilisation d'au moins 0,1% en poids d'agents gem-tensioactifs anioniques de formule générale I, caractérisé en ce que l'on utilise comme composés tensioactifs cationiques des sels d'alkyl triméthylammonium, des sels de dialkyldiméthyl ammonium, des sels d'alkyldiméthylbenzylammonium, des dérivés d'imidazolinium, des sels d'alkylpyridinium, des esters d'acides gras quaternisés d'alcanolamines, des sels d'alkylisoquinolinium, des chlorures de benzéthonium et des dérivés d'acides acylaminés cationiques.

10. Agent selon la revendication 6, avec utilisation d'au moins 0,1% en poids d'agents gem-tensioactifs anioniques de formule générale I, caractérisé en ce que l'on utilise comme bétaïnes et ampholytes tensioactives de la bétaïne de cocosalicylamidopropyle, de la bétaïne d'acylamidopentanediéthyle, de la bétaïne de diméthylammoniohexanoate-acylamidopropane-(ou éthane-)diméthyle (ou diéthyle) avec des longueurs de chaîne de carbone de 10 à 18 atomes de carbone, des sulfobétaïnes, des dérivés d'imidazoline, des lipides d'huile de soja et de la lécithine et leurs mélanges.

11. Agent de nettoyage pour textiles avec utilisation d'au moins 0,1% en poids d'agents gem-tensioactifs anioniques de formule générale I selon l'une quelconque des revendications précédentes.

12. Agent de nettoyage pour surfaces dures avec utilisation d'au moins 0,1% en poids d'agents gem-tensioactifs anioniques de formule générale I selon l'une quelconque des revendications précédentes.

13. Agent de rinçage manuel avec utilisation d'au moins 0,1% en poids d'agents gem-tensioactifs anioniques de formule I selon l'une quelconque des revendications précédentes.

14. Agent de nettoyage pour la peau et les cheveux avec utilisation d'au moins 0,1% d'agents gem-tensioactifs anioniques de formule générale I selon l'une quelconque des revendications précédentes.

15. Agent de soins corporels avec utilisation d'au moins 0,1% en poids d'agents gem-tensioactifs anioniques de formule générale I selon l'une quelconque des revendications précédentes.
